# EUROPÄISCHE PATENTANMELDUNG

(11) **EP 4 368 172 A1**
(43) Veröffentlichungstag der Anmeldung: **15.05.2024**
(21) Anmeldenummer: 22207009.6
(22) Anmeldetag: 11.11.2022
(51) Int. Cl.: A61K 9/20, A61K 41/00, A61K 36/81, B01J 19/08, A61K 36/28, A61K 36/00

(54) **VERFAHREN ZUR HERSTELLUNG EINES ANALOGEN HOMÖOPATHISCHEN WIRKSTOFFS, VORRICHTUNG ZUR DURCHFÜHRUNG DES VERFAHRENS UND COMPUTERPROGRAMMPRODUKT**

(71) Anmelder: Overbeck, Gernot A., 4059 Basel (CH)
(72) Erfinder: Overbeck, Gernot A., 4059 Basel (CH)
(74) Vertreter: Patentanwälte und Rechtsanwalt Weiß, Arat & Partner mbB

(57) **Zusammenfassung**

Ein Verfahren zur Herstellung eines analogen homöopathischen Wirkstoffes aus einer Trägersubstanz soll folgende Schritte umfassen:
- Bereitstellen der Trägersubstanz
- Dynamisierung der Trägersubstanz umfassend ein Aufbringen einer elektromagnetischen Strahlung und/oder ein Aufbringen von Schallwellen auf die Trägersubstanz.

## Beschreibung

### Technisches Gebiet

Die Erfindung betrifft ein Verfahren zur Herstellung eines analogen homöopathischen Wirkstoffes, eine Vorrichtung zur Durchführung dieses Verfahrens, einen nach dem Verfahren hergestellten analogen homöopathischen Wirkstoff sowie ein Computerprogrammprodukt.

### Stand der Technik

Homöopathische Wirkstoffe sind seit den Zeiten Samuel Hahnemanns bekannt und gebräuchlich. Zur Behandlung einer Erkrankung werden homöopathische Wirkstoffe eingesetzt, die durch wiederholte Verdünnung beispielsweise aus einer Urtinktur hergestellt wurden. Oft enthält die Urtinktur einen Wirkstoff, der bei unverdünnter Einnahme die Symptome der zu behandelnden Krankheit hervorruft. Durch wiederholtes Verdünnen und Verschütteln dieses Wirkstoffs und, falls er nicht in flüssiger Form verabreicht wird, anschliessendes Aufbringen auf Globuli oder dergleichen, wird der homöopathische Wirkstoff hergestellt. Das Aufbringen des Wirkstoffs auf Globuli und dergleichen wird "Imprägnieren" genannt. Urtinkturen enthalten beispielsweise Pflanzengifte wie Belladonna, hochgiftige Schwermetalle wie Arsen oder Quecksilber oder Reinkulturen krankmachender Bakterien wie Streptokokken in hoher Konzentration. Eine zu geringe Verdünnung bei der Herstellung des homöopathischen Wirkstoffs birgt also die Gefahr ernsthafter Erkrankungen.

Das Verdünnen und Verschütteln, oft als "Potenzieren" oder "Dynamisieren" bezeichnet, umfasst mehrere Verdünnungsschritte im Verhältnis 1:10 bei sogenannten D-Potenzen, im Verhältnis 1:100 bei sogenannten C-Potenzen und im Verhältnis von 1:50'000 bei sog. Q-Potenzen. Hierbei werden, je nach Potenz, bei jedem Verdünnungsschritt 10 oder 100 Schüttelschläge durchgeführt.

Homöopathische Kombinations-Präparate umfassend zumindest zwei homöopathische Wirkstoffe existieren zwar, allerdings handelt es sich bei vielen homöopathischen Wirkstoffen um Einzelpräparate umfassend nur einen einzigen homöopathischen Wirkstoff. Ein Kombinations-Präparat umfassend mehrere klassische homöopathische Wirkstoffe verschiedener Potenzen, beispielsweise einer D-Potenz und einer C-Potenz, ist nur herstellbar, indem beide Wirkstoffe separat hergestellt und dann vermischt werden. Die verschiedenen Verdünnungsverhältnisse machen eine gemeinsame Herstellung in einem einzigen Fläschchen unmöglich.

### Aufgabe der Erfindung

Die Aufgabe der vorliegenden Erfindung ist es, die Nachteile aus dem Stand der Technik zu überwinden.

### Lösung der Aufgabe

Zur Lösung der Aufgabe führen die Gegenstände der unabhängigen Ansprüche. Vorteilhafte Ausgestaltungen sind in den abhängigen Ansprüchen beschrieben.

Ein Verfahren gemäss der vorliegenden Erfindung zur Herstellung eines analogen homöopathischen Wirkstoffes aus einer Trägersubstanz umfasst folgende Schritte:
- Bereitstellen der Trägersubstanz und
- Dynamisierung der Trägersubstanz umfassend ein Aufbringen einer elektromagnetischen Strahlung und/oder ein Aufbringen von Schallwellen auf die Trägersubstanz.

Im Rahmen der vorliegenden Erfindung werden auch nachstehend noch näher beschriebene Globuli, Salben und dergleichen unter dem Begriff "Wirkstoff" subsumiert. Letzterer umfasst also beliebige Darreichungsformen und ist im Gegensatz zur schulmedizinischen Definition, wonach beispielsweise eine Tablette üblicherweise nur zu einem Teil aus Wirkstoff besteht, breiter gefasst.

Wie nachstehend noch erläutert ist, meint das Aufbringen hierbei vorzugsweise ein gerichtetes und gezieltes Aufbringen, vorzugsweise mit zumindest einer nahe der Trägersubstanz platzierten Strahlungs- und/oder Schallquelle.

Im Gegensatz zur Dynamisierung klassischer homöopathischer Wirkstoffe umfassend das Verdünnen und Verschütteln der Ursubstanz, umfasst die Dynamisierung des analogen homöopathischen Wirkstoffs gemäss der vorliegenden Erfindung das Aufbringen der elektromagnetischen Strahlung (nachfolgend "Strahlung") und/oder der Schallwellen auf die Trägersubstanz. Da keine giftigen Urtinkturen verwendet werden, besteht bei dem erfindungsgemässen Verfahren auch kein Risiko, versehentlich Giftstoffe in zu hoher Konzentration in den homöopathischen Wirkstoff einzuschleppen, beispielsweise wegen zu niedrig gewählter Verdünnung oder einem nicht ordnungsgemäss durchgeführten Verdünnungs-Vorgang.

Der analoge homöopathische Wirkstoff unterscheidet sich von einem klassischen homöopathischen Wirkstoff vorzugsweise ausschliesslich durch die Art der Dynamisierung, also dadurch, wie die Dynamisierung erfolgt. Insbesondere können bei analogen homöopathischen Wirkstoffen dieselben Trägersubstanzen eingesetzt werden, wie bei klassischen homöopathischen Wirkstoffen.

Als Trägersubstanz kommen beispielsweise Globuli, Tabletten, Salben oder Flüssigkeiten in Betracht. Es kann daran gedacht sein, diese Trägersubstanzen in unbehandelter Form einzusetzen. Eine unbehandelte Trägersubstanz wurde vor der Dynamisierung gemäss der vorliegenden Erfindung nicht mit einem schulmedizinischen oder homöopathischen Wirkstoff versehen.

Alternativ kann daran gedacht sein, jedenfalls solche Trägersubstanzen einzusetzen, welche nicht mit einem homöopathischen Wirkstoff versehen wurden. Es kann also angedacht sein, schulmedizinische Arzneimittel gemäss der vorliegenden Erfindung zu dynamisieren. Insbesondere kann an schulmedizinische Arzneimittel in Tablettenform gedacht sein.

Weiter alternativ können als Trägersubstanzen Nahrungs- oder Genussmittel in Betracht kommen, beispielsweise in Form von Bonbons, beispielsweise Hustenbonbons, oder dergleichen.

Herstellung und Zusammensetzung der genannten Trägersubstanzen sind dem Fachmann geläufig. Globuli können beispielsweise überwiegend aus Saccharose oder Xylit bestehen. Tabletten und Salben können aus den für diese Darreichungsformen gebräuchlichen Grundsubstanzen hergestellt werden, dasselbe gilt für die anderen oben genannten Darreichungsformen. Als Grundsubstanzen sind unter anderem Wasser, Alkohol, verschiedene Zucker und verschiedene Polymere bekannt.

Vorzugsweise wurde die im Rahmen der vorliegenden Erfindung genutzte Trägersubstanz zuvor nicht durch Verdünnen und Verschütteln behandelt und auch nicht mit einem durch Verdünnen und Verschütteln hergestellten Stoff oder Stoffgemisch behandelt, versetzt, oder vermischt. Wie bereits angedeutet wird als Trägersubstanz besonders bevorzugt ein Stoff eingesetzt, welcher zuvor nicht gemäss den Vorschriften der klassischen Homöopathie, zu denen insbesondere Verschütteln und Verdünnen gehört, behandelt oder mit einem derart behandelten Stoff oder Stoffgemisch behandelt, versetzt, oder vermischt wurde. Die Trägersubstanz ist also vorzugsweise unbehandelt.

Ein analoger homöopathischer Wirkstoff gemäss der vorliegenden Erfindung kann in verschiedenen Darreichungsformen hergestellt werden, beispielsweise in Form von Globuli, Tabletten, Bonbons, Ampullen, Injektionslösungen, Salben, Tropfen, etc.

Das Bereitstellen der Trägersubstanz kann ein Ausbreiten oder Ausleeren der Trägersubstanz auf einer für die Dynamisierung geeigneten Unterlage oder Oberfläche, oder ein Bereitstellen in einem geeigneten offenen oder zumindest teilweise verschlossenen Gefäss umfassen.

Beispielsweise können Globuli, Bonbons oder Tabletten, insbesondere unbehandelte Globuli, Bonbons oder Tabletten, welche als Trägersubstanz eingesetzt werden sollen, in einer Schale, Schüssel oder dergleichen ausgebreitet werden, um anschliessend von der Strahlung und/oder den Schallwellen beaufschlagt zu werden. Dieses Ausbreiten kann das Erzeugen einer einlagigen Schicht aus Globuli oder Tabletten umfassen, so dass sämtliche zu dynamisierenden Globuli oder Tabletten in ausreichendem Umfang mit der Strahlung und/oder den Schallwellen beaufschlagt werden. Auch Flüssigkeiten oder Salben, welche als Trägersubstanz verwendet werden, können in einer Schale, Schüssel oder dergleichen verteilt werden, wie vorstehend für Globuli und Tabletten beschrieben.

Eine zu dynamisierende Flüssigkeit kann in einem geeigneten Gefäss gelagert werden. Das Gefäss kann eine Öffnung umfassen, durch welche die Flüssigkeit mit der Strahlung und/oder den Schallwellen beaufschlagt wird. Das Gefäss kann auch zumindest teilweise aus einem für die Strahlung und/oder die Schallwellen zumindest teilweise durchlässigen Werkstoff gefertigt sein, um ein Eindringen der Strahlung und/oder der Schallwellen in die Flüssigkeit durch die Wände des Gefässes zu erlauben. Als Gefäss kommen hierbei Flaschen, Gläser, Becher, aber auch Schüsseln oder andere Gefässe in Betracht. Derartige Gefässe wie beispielsweise Flaschen, Gläser, oder dergleichen, können beispielsweise auch zu dynamisierende Salben, Globuli, Tabletten oder Bonbons aufnehmen.

Eine zu dynamisierende Salbe kann auf dieselbe Weise dynamisiert werden, wie dies vorstehend für Flüssigkeiten und Globuli oder Tabletten beschrieben wurde. Eine Salbe kann beispielsweise in einem Gefäss gelagert und dort dynamisiert werden. Alternativ kann die Salbe auf einer geeigneten Unterlage zu einer dünnen Schicht ausgestrichen werden, um eine möglichst intensive Beaufschlagung mit der Strahlung und/oder den Schallwellen zu ermöglichen. Auch Globuli, Tabletten oder Bonbons können auf einfache Weise auf einer geeigneten Unterlage, beispielsweise einer Tischoberfläche, ausgebreitet werden, um dort dynamisiert zu werden.

Wie bereits erwähnt erfolgt die Herstellung des analogen homöopathischen Wirkstoffs vorzugsweise ohne ein Verdünnen und ohne ein Verschütteln.

Das Aufbringen der Strahlung kann durch Bestrahlung der Trägersubstanz mittels einer geeigneten Strahlungsquelle über einen bestimmten Zeitraum, d.h. über eine bestimmte Bestrahlungsdauer hinweg erfolgen. Beispiele hierfür werden nachstehend näher erläutert.

Das Aufbringen der Schallwellen kann durch Beschallen mittels einer geeigneten Schallquelle über einen bestimmten Zeitraum, d.h. über eine bestimmte Beschallungsdauer hinweg erfolgen. Beispiele hierfür werden ebenfalls nachstehend erläutert.

Für das Aufbringen der Strahlung können eine oder mehrere Strahlungsquellen eingesetzt werden. Für das Aufbringen der Schallwellen können eine oder mehrere Schallquellen eingesetzt werden. Die vorgenannten Quellen können gleichzeitig oder nacheinander die jeweils ausgesandte Strahlung und/oder die ausgesandten Schallwellen auf die Trägersubstanz aufbringen.

Die Dynamisierung der Trägersubstanz kann aus dem Aufbringen der Strahlung und/oder der Schallwellen auf die Trägersubstanz bestehen. Es kann also daran gedacht sein, dass die Dynamisierung keine weiteren Schritte umfasst.

Die Strahlung kann ausgewählt sein aus der Gruppe des für den Menschen sichtbaren Lichts (nachfolgend "sichtbares Licht"), UV-Strahlung und Infrarot-Strahlung. Vorzugsweise ist die Strahlung ausgewählt aus der Gruppe umfassend sichtbares Licht und Infrarot-Strahlung; noch bevorzugter handelt es sich bei der Strahlung um sichtbares Licht. Das Dynamisieren kann ein Bestrahlen der Trägersubstanz mit zumindest einer der vorgenannten Strahlungsarten umfassen.

Schallwellen sind im Allgemeinen sich in einem Medium ausbreitende Druck- und Dichteschwankungen. Bei den Schallwellen kann es sich um Töne handeln, also um Schallereignisse, welche in der menschlichen Sinneswahrnehmung als Ton wahrgenommen werden.

Sowohl die Strahlung als auch die Schallwellen können durch verschiedene Parameter charakterisiert werden, beispielsweise Frequenz, Amplitude sowie Bestrahl- und/oder Beschalldauer:
Die Frequenz kann mit der Farbe und/oder der Tonhöhe korrelieren.

Die Amplitude kann mit der Lichtstärke und/oder der Lautstärke eines Tons korrelieren.

Eine Bestrahl- oder Beschalldauer kann einen Zeitraum definieren, über welchen hinweg die Bestrahlung oder Beschallung erfolgt.

Frequenz, Amplitude und Bestrahldauer können eine Strahlung physikalisch ausreichend beschreiben. Üblicherweise wird jedoch, so meist auch im Rahmen der vorliegenden Erfindung, beispielsweise kein monochromatisches Licht eingesetzt. Farben sind letztlich Sinneswahrnehmungen des Menschen, welche durch Wechselwirkung zwischen der Strahlung und den Sehzellen sowie der nachfolgenden Verarbeitung im Gehirn entstehen. Da derselbe Farbeindruck auf verschiedene Weisen, beispielsweise durch subtraktive oder additive Farbmischung oder durch monochromatisches Licht hervorgerufen werden kann, ist eine eineindeutige Zuordnung einer Farbe zu einer bestimmten Frequenz nicht möglich. Im Rahmen der vorliegenden Erfindung ist daher auch die Farbe im Sinne eines Farbeindrucks beim Menschen sowie der Ton im Sinne einer vom Menschen wahrgenommenen Tonhöhe als Parameter anzusehen.

Auch die Polarisation der Strahlung und die Energie der Strahlung oder der Schallwellen können als Parameter angesehen werden.

Analoge Überlegungen gelten für die Schallwellen, wobei im Rahmen der vorliegenden Erfindung anstatt eines Tons ein aus mehreren Tönen und gegebenenfalls Obertönen zusammengesetzter Klang zum Einsatz kommen kann.

Im Rahmen der vorliegenden Erfindung werden eine Farbe der Strahlung, also der von ihr hervorgerufene Sinneseindruck, und eine Tonhöhe der Schallwellen, also der von ihnen hervorgerufene Sinneseindruck, auch als "Parameter" bezeichnet. Farbe und Tonhöhe sind die bevorzugten Parameter, welche die Strahlung und/oder die Schallwellen charakterisieren. Im Falle einer Sequenz ändert sich in jedem Zeitabschnitt im Vergleich zum vorigen und/oder nachfolgenden Zeitabschnitt meist zumindest einer der folgenden Parameter: Frequenz, Amplitude, Farbe, Tonhöhe, Polarisation. Die Bestrahl- und/oder Beschalldauer jedes Zeitabschnitts einer Frequenz kann ebenfalls variieren.

Üblicherweise umfassen eine als Farbe wahrgenommene Strahlung und ein als Ton wahrgenommener Schall ein Frequenzspektrum, sind also jeweils aus unterschiedlichen Strahlungen oder Tönen mit bestimmter Frequenz und Amplitude zusammengesetzt oder können zumindest als solche gedacht sein.

Die Frequenz der Strahlung oder die Frequenzen ihres Frequenzspektrums können zwischen 380 und 790 THz oder zwischen 401 und 789 THz liegen, was ungefähr Wellenlängen von 380 bis 750 nm entspricht.

Die Frequenz der Schallwellen oder die Frequenzen ihres Frequenzspektrums können zwischen 10 Hz und 25 kHz, vorzugsweise zwischen 15 Hz und 20 kHz, beispielsweise zwischen 20 Hz und 20 kHz liegen.

Im Allgemeinen kann die Strahlung geeignet sein, vom Menschen als Farbe wahrgenommen zu werden. Im Allgemeinen können die Schallwellen geeignet sein, vom Menschen als Ton wahrgenommen zu werden. Besteht die Strahlung nicht aus monochromatischem Licht und/oder liegt statt eines Tons ein Klang vor, so kann im Allgemeinen daran gedacht sein, dass zumindest ein Teil der Strahlung und/oder ein Teil der Töne vom Menschen als Farbe und/oder Klang oder Ton wahrgenommen werden kann.

Es kann daran gedacht sein, dass die Strahlung und/oder die Schallwellen in einer Sequenz aufgebracht werden, wobei eine solche Sequenz zumindest zwei Zeitabschnitte umfasst, während denen die Strahlung und/oder die Schallwellen aufgebracht werden, und wobei sich die Strahlung und/oder die Schallwellen, welchen in diesen Zeitabschnitten aufgebracht werden, im Hinblick auf zumindest einen oben beschriebenen Parameter unterscheiden.

Jeder der vorgenannten Zeitabschnitte kann durch die oben bereits angegebenen Parameter charakterisiert sein, nämlich Frequenz, Amplitude, Bestrahl- und/oder Beschalldauer, Farbe, Polarisation oder Tonhöhe der Strahlung und/oder der Schallwellen.

Es kann auch daran gedacht sein, die Energie der Schallwellen oder der Strahlung, manchmal auch als Intensität bezeichnet, als Parameter heranzuziehen. Diese Energie ist wiederum abhängig von der Frequenz und der Amplitude.

Im Rahmen der vorliegenden Erfindung wird der Einfachheit vorwiegend von "Strahlung" und "Schallwellen" gesprochen, wobei die entsprechenden Sequenzen jeweils mit umfasst sind.

Die Strahlung einer Sequenz kann sich in den Zeitabschnitten beispielsweise hinsichtlich des Parameters "Farbe" unterscheiden, für die Schallwellen einer Sequenz gilt entsprechendes für den Parameter "Tonhöhe". Ferner sind im Allgemeinen Unterschiede hinsichtlich Frequenz und/oder Amplitude denkbar.

Die Schallwellen einer Sequenz können sich in den Zeitabschnitten beispielsweise hinsichtlich des "Parameters" Ton bzw. Tonhöhe unterscheiden. Ferner sind Unterschiede hinsichtlich Frequenz und/oder Amplitude denkbar.

Wird im Rahmen von Ausführungsformen der vorliegenden Erfindung von "Schallwellen" oder "Strahlung" gesprochen, so ist stets auch eine vorbeschriebene Sequenz mit umfasst.

Je nach Anzahl und Beschaffenheit der eingesetzten Strahlungs- und Schallquellen kann daran gedacht sein, dass die zumindest eine Strahlungsquelle beispielsweise gleichzeitig mehrere Farben aussendet und/oder dass die zumindest eine Schallquelle beispielsweise einen Klang anstatt eines Tons ausgibt. Ist im Rahmen der vorliegenden Erfindung von "Strahlung" oder "Schallwellen" die Rede, so sollen die vorbeschriebenen Situationen stets mit umfasst sein.

Im Allgemeinen kann die Dynamisierung allein durch Beschallung mit den Schallwellen oder allein durch Bestrahlung mit der Strahlung erfolgen. Es kann auch daran gedacht sein, dass Beschallung und Bestrahlung gleichzeitig oder abwechselnd erfolgen. Ferner kann auch an eine Dynamisierung gedacht sein, welche ausschliesslich durch Beschallung oder ausschliesslich durch Bestrahlung erfolgt.

Das Verfahren kann die folgenden Schritte umfassen:
- die Strahlung und/oder die Schallwellen, welche durch zumindest einen Parameter charakterisiert sind, werden für die Dynamisierung ausgewählt;
- die Dynamisierung der Trägersubstanz erfolgt durch Aufbringen der Strahlung und/oder der Schallwellen, welche durch den zumindest einen Parameter charakterisiert sind.

Es kann also zunächst eine Auswahl und anschliessend die Dynamisierung stattfinden.

Vor den beiden vorgenannten Schritten kann dem analogen homöopathischen Wirkstoff der zumindest eine Parameter der Strahlung und/oder der Schallwellen zugeordnet werden. Es kann also daran gedacht sein, dass die zur Dynamisierung der Trägersubstanz und somit zur Herstellung des analogen homöopathischen Wirkstoffs einzusetzenden Parameter wie beispielsweise Frequenz, Amplitude, Bestrahl- und/oder Beschalldauer, Farbe oder Tonhöhe der Schallwellen und/oder der Strahlung im Vorfeld festgelegt wurden.

Die Zuordnung kann derart erfolgen, dass für eine Vielzahl herzustellender analoger homöopathischer Wirkstoffe jeweils zumindest einer, meist jedoch mehrere der o.g. Parameter festgelegt und beispielsweise in Form einer Liste abgelegt werden. Weiterhin sind bevorzugt die weiter unten besprochenen Eingaben eines Benutzers ebenfalls dem oder den Parametern und vorzugsweise gleichzeitig auch dem entsprechenden analogen homöopathischen Wirkstoff zugeordnet und werden entsprechend in der Liste abgelegt.

Wird, wie nachstehend noch ausführlich beschrieben, eine App oder im Allgemeinen ein Computerprogrammprodukt zur Umsetzung des Verfahrens benutzt, so kann daran gedacht sein, dass die Liste in Form einer Lookup-Tabelle vorliegt, auf welche die App oder das Computerprogrammprodukt zugreift. Lookup-Tabellen, auch Umsetzungstabellen genannt, umfassend statisch definierte Einträge.

Abhängig von Anzahl und Kombinationsmöglichkeiten der (weiter unten im Detail beschriebenen) Eingaben des Benutzers, der Parameter und der analogen homöopathischen Wirkstoffe kann eine solche Liste sehr umfangreich werden, also eine sehr grosse Anzahl statisch festgehaltener Zuordnungen umfassen.

Anstelle einer derart statisch festgelegten Zuordnung kann auch daran gedacht sein, dass die Zuordnung im Wege einer Zuordnungsvorschrift erfolgt. Wird im Rahmen der vorliegenden Erfindung von der Zuordnung gesprochen, so ist stets eine solche Zuordnungsvorschrift mit umfasst.

Der grundsätzlichen Arbeitsweise eines Computerprogramms folgend, welches nacheinander die Schritte Eingabe, Verarbeitung, Ausgabe umfasst, kann die Zuordnungsvorschrift die Verarbeitung festlegen. Die Eingabe kann die weiter unten ausführlich beschriebene Eingabe eines Benutzers sein, auf der basierend die Strahlung und/oder die Schallwellen ausgewählt werden. Die Schallwellen und/oder Strahlung, mit welcher die Trägersubstanz beaufschlagt wird/werden, stellen die Ausgabe dar.

Die Zuordnungsvorschrift kann so beschaffen sein, dass sie für dieselbe Eingabe eines Benutzers reproduzierbar stets dieselben oder denselben Parameter und/oder denselben analogen homöopathischen Wirkstoff ausgibt. Gibt der Benutzer beispielsweise zumindest ein körperliches Symptom und/oder zumindest einen klassischen homöopathischen Wirkstoff ein, so kann die Zuordnungsvorschrift darauf basierend reproduzierbar und eindeutig Angaben zur Strahlung und/oder den Schallwellen liefern, welche zur Herstellung eines analogen homöopathischen Wirkstoffs benötigt werden. Auf diese Weise ist es problemlos möglich, einen analogen homöopathischen Komplex-Wirkstoff herzustellen, indem der Benutzer mehrere klassische homöopathische Wirkstoffe eingibt, welche durch die Zuordnungsvorschrift in einen einzigen analogen homöopathischen Wirkstoff "übersetzt" werden.

Wie nachfolgend noch näher erläutert wird, kann das Verfahren auf einem mit Computer-Funktionalitäten ausgestatteten Gerät, beispielsweise einem Smartphone, ausgeführt werden. Hierfür kann das Smartphone mit einem Computerprogrammprodukt, beispielsweise einer sogenannten App, ausgestattet sein. Diese App kann auf dem Smartphone installiert sein.

Die vorstehend beschriebene Zuordnung kann bei der Entwicklung der App oder im Allgemeinen des Computerprogrammprodukts und/oder in Form von Updates der App oder des Computerprogrammprodukts erfolgen. Die Zuordnung kann beispielsweise durch einen Homöopathen erfolgen, der sich hierfür seiner Erfahrung und Fachkenntnis bedienen kann.

Erfolgte diese Zuordnung nicht unmittelbar vor der Dynamisierung der Trägersubstanz, sondern zuvor mit deutlichem zeitlichen Abstand, und wurde diese Zuordnung durch Ablegen, Abspeichern etc. festgehalten, so kann nach der Auswahl und unmittelbar vor der Dynamisierung statt einer Zuordnung eine Abfrage der bereits im Vorfeld erfolgten Zuordnung erfolgen.

Insbesondere bei der Herstellung des analogen homöopathischen Wirkstoffs durch beispielsweise einen Homöopathen kann die Zuordnung unmittelbar vor der Dynamisierung und einem Ausführungsbeispiel gemäss auch gleichzeitig mit der Auswahl stattfinden.

Wendet ein nicht Homöopathie-kundiger Benutzer das Verfahren beispielsweise in seinen Wohnräumen an, um beispielsweise mittels des Verfahrens eine kleine Menge des homöopathischen Wirkstoffs für sich oder für jemanden aus seinem näheren Umfeld herzustellen, so wird die Zuordnung vorzugsweise mit deutlichem zeitlichen Abstand im Vorfeld erfolgt sein.

In beiden vorgenannten Fällen, d.h. unabhängig vom zeitlichen Abstand zwischen der Zuordnung und der Dynamisierung, kann die Auswahl der Strahlung und/oder Schallwellen, welche durch zumindest einen Parameter charakterisiert sind, indirekt erfolgen: Durch die Zuordnung können der Benutzer, beispielsweise ein Patient, oder der Homöopath anstatt des zumindest einen Parameters, beispielsweise der Farbe der Strahlung, unmittelbar den gewünschten analogen homöopathischen Wirkstoff auswählen. Auf Grund der Zuordnung wird durch die Auswahl des Wirkstoffs mittelbar der zumindest eine Parameter ausgewählt.

Alternativ kann die Auswahl auch direkt erfolgen, indem insbesondere ein in der Homöopathie kundiger Benutzer den zumindest einen Parameter auswählt, also in irgendeiner Form vorgibt.

Im Folgenden sind von dem Begriff "Benutzer" beliebige Benutzer umfasst, also sowohl beispielsweise Patienten und deren Angehörige, welche für sich oder die Angehörigen einen analogen homöopathischen Wirkstoff herstellen wollen, als auch ein Homöopath, der den Wirkstoff beispielsweise zum Verkauf herstellen möchte.

Der herzustellende analoge homöopathische Wirkstoff kann dem Benutzer in einer Liste zur Auswahl präsentiert werden, welche eine Mehrzahl von analogen homöopathischen Wirkstoffen umfasst. Beispielsweise kann daran gedacht sein, dass der Benutzer aus einer Liste, welche ihm angezeigt wird, den herzustellenden homöopathischen Wirkstoff auswählt. Im Allgemeinen kann daran gedacht sein, dass der Benutzer den analogen homöopathischen Wirkstoff in irgendeiner Weise auswählt. Wie bereits beschrieben erfolgte vorzugsweise zuvor bereits die Zuordnung des zumindest einen Parameters, welcher die Schallwellen und/oder die Strahlung charakterisiert, zu dem analogen homöopathischen Wirkstoff. Somit bestimmt der Benutzer durch die Auswahl des vorgenannten Wirkstoffs gleichzeitig auch die Strahlung und/oder die Schallwellen, welche durch den zumindest einen Parameter charakterisiert sind.

Anstatt den alternativen homöopathischen Wirkstoff aus einer Liste auszuwählen, kann alternativ daran gedacht sein, dass der Benutzer den herzustellenden homöopathischen Wirkstoff durch Eingaben vorgibt und gestaltet:
Bei dem weiter oben beschriebenen Verfahren, bei welchem zunächst die Auswahl der Strahlung und/oder der Schallwellen durch direkte oder indirekte Auswahl zumindest eines Parameters und anschliessend die Dynamisierung erfolgt, kann insbesondere der Auswahl das Empfangen einer Eingabe eines Benutzers vorangestellt sein.

Eine solche Eingabe kann eine Information umfassen, welche unmittelbar den herzustellenden analogen homöopathischen Wirkstoff betrifft. Dies kann erfolgen, indem die Bezeichnung des vorgenannten Wirkstoffs in ein entsprechendes Eingabefeld eingetippt oder aus einer dem Benutzer präsentierten Liste ausgewählt wird. Weiterhin kann in derselben Weise der zur Herstellung benötigte Parameter der Strahlung und/oder der Schallwellen eingegeben werden. Beide vorgenannten Fälle wurden weiter oben bereits erläutert.

Im Allgemeinen kann das Verfahren also folgende Schritte umfassen:
- Empfangen einer Eingabe eines Benutzers, die Eingabe umfassend zumindest eine Information,
- die elektromagnetische Strahlung und/oder die Schallwellen, welche durch zumindest einen Parameter charakterisiert sind, werden basierend auf der Eingabe des Benutzers für die Dynamisierung ausgewählt.

Weiterhin kann daran gedacht sein, dass die Eingabe eine Information umfasst, welche zumindest einen klassischen oder analogen homöopathischen Wirkstoff und/oder zumindest eine Verdünnung und/oder zumindest ein körperliches Symptom und/oder zumindest einen Namen einer Zubereitung umfassend einen klassischen homöopathischen Wirkstoff und/oder zumindest einen Namen eines Herstellers einer solchen Zubereitung betrifft. Auch in einem solchen Fall kann daran gedacht sein, dass dem Benutzer eine Liste umfassend die möglichen Eingaben präsentiert wird. Hierfür können beispielsweise Listen mit einer Vielzahl der vorstehend genannten Eingaben, beispielsweise klassischen homöopathischen Wirkstoffen und Verdünnungen sowie körperlichen Symptomen und die vorgenannten Namen zur Präsentation bereitgestellt werden. Die vorstehend erwähnte "Verdünnung" meint vorzugsweise eine D-, C-, oder Q-Potenz.

Der oben erwähnte Name einer Zubereitung kann ein Handelsname, ein Markenname oder eine Fachbezeichnung eines klassischen homöopathischen Arzneimittels sein, oder einer Zubereitung umfassend neben anderen Bestandteilen einen klassischen homöopathischen Wirkstoff.

Umfasst die Eingabe eine Information betreffend einen klassischen homöopathischen Wirkstoff, so umfasst sie vorzugsweise auch eine Verdünnung. Klassische homöopathische Arzneimittel werden in der Regel durch den Wirkstoff und die Verdünnung beschrieben.

Das oben genannte körperliche Symptom kann ein Beschwerdesymptom eines Lebewesens, also beispielsweise eines Menschen oder eines Tieres sein.

Es kann explizit daran gedacht sein, dass die Eingabe Informationen betreffend sowohl zumindest ein körperliches Symptom als auch betreffend beispielsweise einen klassischen homöopathischen Wirkstoff oder einen Namen umfasst. Dies kann vorteilhaft sein, wenn ein Benutzer für bestimmte Beschwerden einen analogen homöopathischen Wirkstoff herstellen möchte, und er weiterhin weiss, dass ihm ein bestimmter klassischer homöopathischer Wirkstoff in der Vergangenheit Abhilfe seiner Beschwerden verschafft hat. Durch die Zuordnungsvorschrift werden die vorgenannten Eingaben dann in Schallwellen oder Strahlung "übersetzt", um den gewünschten analogen homöopathischen Wirkstoff herzustellen.

Gibt der Benutzer ein körperliches Symptom oder eine der anderen vorgenannten Informationen ein, so kann daran gedacht sein, dass ihm eine Mehrzahl passender analoger homöopathischer Wirkstoffe präsentiert werden, aus denen er dann einen Wirkstoff auswählen kann. Auch eine derart gestaltete Auswahl, welche in mehrere Schritte unterteilt ist, ist eine Auswahl basierend auf der Eingabe des Benutzers.

Für sämtliche Ausführungsformen der Verfahren gemäss der vorliegenden Erfindung kann ein mit Computer-Funktionalitäten ausgestattetes Gerät, beispielsweise ein Smartphone, eingesetzt werden. Es kann auch daran gedacht sein, ein System umfassend ein solches Gerät einzusetzen. Die Strahlung und/oder die Schallwellen können also von einem mit Computer-Funktionalitäten ausgestatteten Gerät, beispielsweise von einem Smartphone, oder einem System umfassend ein solches Gerät auf die Trägersubstanz aufgebracht werden.

Wird ein System eingesetzt, so umfasst dieses zumindest ein Computer-Funktionalitäten ausgestattetes Gerät.

Das vorgenannte Gerät oder System umfasst vorzugsweise eine Schnittstelle zur Interaktion mit dem Benutzer. Dies kann beispielsweise ein berührungsempfindlicher Bildschirm, beispielsweise ein Smartphone-Bildschirm sein.

Das vorgenannte Gerät oder System umfasst vorzugsweise eine Schallquelle und/oder eine Strahlungsquelle. Die Schallquelle kann insbesondere ein Lautsprecher sein, mit dem die Schallwellen auf die Trägersubstanz aufgebracht werden können. Die Strahlungsquelle kann beispielsweise ein beliebiges Leuchtmittel sein, mit dem die Strahlung auf die Trägersubstanz aufgebracht werden kann. Hierbei kann auch an einen Bildschirm, beispielsweise einen Smartphone-Bildschirm als Strahlungsquelle gedacht sein.

Das vorgenannte Gerät oder System umfasst vorzugsweise einen Speicher oder hat Zugriff auf einen externen Speicher, welcher sich beispielsweise in einem Rechnernetzwerk (Cloud Computing) befinden kann. Die oben beschriebenen Zuordnungen können in diesem Speicher abgelegt sein.

Das vorgenannte Gerät oder System umfasst vorzugsweise Mittel zum Präsentieren, um beispielsweise eine dem Benutzer die weiter oben beschriebene Liste zur Auswahl zu präsentieren. Dieses Mittel kann beispielsweise der berührungsempfindliche Bildschirm, beispielsweise der Smartphone-Bildschirm sein. Die Schnittstelle und das Mittel zum Präsentieren können also in einem Bauteil zusammengefasst sein. Alternativ kann auch an einen nicht berührungsempfindlichen Bildschirm beispielsweise zum Präsentieren gedacht sein, wobei die Schnittstelle in anderer Form, beispielsweise durch Knöpfe oder andere Bedienelemente im Gerät oder System realisiert sein können.

Auf dem mit Computer-Funktionalitäten ausgestatteten System kann vorzugsweise das nachfolgend noch beschriebene Computerprogrammprodukt ausgeführt werden.

Bei sämtlichen Ausführungsformen der vorliegenden Erfindung kann es sich bei dem vorstehend genannten System oder Gerät um ein tragbares System oder Gerät handeln. Beispielsweise kann es sich um ein Smartphone oder ein anderes mit einem Bildschirm ausgestattetes tragbares Gerät, beispielsweise ein Tablet oder dergleichen handeln. Ein Bildschirm, insbesondere ein Smartphone-Bildschirm, kann die Strahlung bereitstellen, mit der die Dynamisierung erfolgt.

Der Smartphone-Bildschirm kann die einzige Strahlungsquelle sein, welche gemäss dem Verfahren zur Bestrahlung der Trägersubstanz verwendet wird.

Alternativ kann es sich um ein System handeln, welches unter anderem eine Strahlungsquelle zum Bereitstellen der Strahlung für die Dynamisierung und andererseits ein mit der Strahlungsquelle verbundenes und mit Computer-Funktionalitäten ausgestattetes Gerät umfasst.

Ein oder mehrere Lautsprecher eines Smartphones kommen entsprechend als Schallquelle(n) in Betracht.

Alternativ kann es sich um ein System handeln, welches unter anderem eine Schallquelle zum Bereitstellen der Schallwellen für die Dynamisierung und andererseits ein mit der Schallquelle verbundenes und mit Computer-Funktionalitäten ausgestattetes Gerät umfasst.

Die mit dem Verfahren gemäss der vorliegenden Erfindung herstellbaren Wirkstoffe erweitern die Gesamtheit der bislang verfügbaren klassischen homöopathischen Wirkstoffe. Neben letzteren umfassen die mit Ausführungsformen des erfindungsgemässen Verfahrens hergestellten analogen homöopathischen Wirkstoffe nämlich beliebige Gemische, welche bislang nicht oder nur auf komplizierte Weise herstellbar waren. Bei dem analogen homöopathischen Wirkstoff kann es sich also beispielsweise einerseits analoge Varianten zu den klassischen homöopathischen Wirkstoffen, also beispielsweise um analoges Belladonna D12, analoges Belladonna C30 oder analoges Arnica D6 handeln. Im Allgemeinen kann es sich also um analoge homöopathische Wirkstoffe handeln, welche auch als klassische homöopathische Wirkstoffe erhältlich sind. Daneben sind zahlreiche Mischungen aus zwei oder mehr dieser Wirkstoffe als analoger homöopathischer Wirkstoff herstellbar.

Die vorliegende Erfindung umfasst ferner eine Vorrichtung, welche eingerichtet ist, sämtliche Varianten des vorstehend beschriebenen Verfahrens durchzuführen. Diese Vorrichtung kann ein mit Computer-Funktionalitäten ausgestattetes Gerät, insbesondere ein Smartphone sein. Allgemein kann es sich um tragbare Geräte handeln.

Die vorliegende Erfindung umfasst ferner ein System umfassend das vorstehend beschriebene Gerät.

Die vorliegende Erfindung umfasst ferner ein System mit einer tragbaren Vorrichtung wie vorstehend beschrieben sowie zumindest einer weiteren Komponente, beispielsweise einer separaten Schallquelle und/oder zumindest einer nachstehend beschriebenen Halterung.

Die vorliegende Erfindung umfasst ferner ein System mit einer tragbaren Vorrichtung wie vorstehend beschrieben sowie mit einer Halterung, welche eingerichtet ist, die tragbare Vorrichtung in einem vorbestimmten Abstand zu einer in ihrer Nähe platzierten Trägersubstanz zu fixieren. Kommt eine separate Schallquelle zum Einsatz, so kann auch für diese eine entsprechende Halterung vorgesehen sein.

Diese Halterung kann beispielsweise ein Gestell oder Stativ sein, welche ein Smartphone derart in einer vorbestimmten Höhe über einem Tisch oder dergleichen fixiert, auf welchem das Gestell oder Stativ steht, dass die bereitgestellte Trägersubstanz durch die Strahlung des Smartphone-Bildschirms bestrahlt und somit dynamisiert werden kann.

Die vorliegende Erfindung umfasst ferner ein Computerprogrammprodukt umfassend Instruktionen, die, wenn sie von einem mit Computer-Funktionalitäten ausgestatteten System oder Gerät ausgeführt werden, bewirken, dass dieses die Schritte gemäss zumindest einer Ausführungsform der eingangs beschriebenen Verfahren durchführt.

Das Gerät kann ein tragbares Gerät, insbesondere ein Smartphone sein, wie vorstehend bereits beschrieben. Das System kann ferner eine separate Schallquelle, beispielsweise einen Bluetooth-Lautsprecher umfassen. Der Bluetooth-Lautsprecher kann mit dem Smartphone gekoppelt sein und somit ebenso durch die App, also das Computerprogrammprodukt, gesteuert werden, und die zu dynamisierende Trägersubstanz mit Schallwellen beaufschlagen.

Die vorliegende Erfindung umfasst ferner einen analogen homöopathischen Wirkstoff, welcher gemäss Ausführungsformen des eingangs beschriebenen Verfahrens hergestellt ist.

Wie bereits beschrieben kann die Dynamisierung der Trägersubstanz erfolgen, indem die für die Bestrahlung nötige(n) Farbe(n) von einem Smartphone-Bildschirm abgestrahlt werden, und die Trägersubstanz mit dieser Strahlung beaufschlagt wird. Der Smartphone-Bildschirm wird hierfür auf die Trägersubstanz gerichtet, was entweder händisch oder mittels eines geeigneten Stativ oder dergleichen erfolgen kann. Es kann auch daran gedacht sein, das Smartphone so auf ein für die Strahlung durchlässiges Gefäss oder dergleichen zu legen, in welchem sich die zu dynamisierende Trägersubstanz befindet, dass der Smartphone-Bildschirm der Trägersubstanz zugewandt ist.

Alternativ oder zusätzlich kann die Trägersubstanz mit Schallwellen beaufschlagt werden, welche aus dem Smartphone-Lautsprecher stammen. Hierbei kann einerseits ein oftmals seitlich am Smartphone angebrachter Lautsprecher zum Einsatz kommen. Im Rahmen der vorliegenden Erfindung wird jedoch auch die Hörmuschel des Smartphones als Lautsprecher aufgefasst. Wird das Smartphone wie weiter oben beschrieben beispielsweise auf das Gefäss oder direkt auf die Trägersubstanz gelegt, so sind in der Regel sowohl die Ohrmuschel als auch der Smartphone-Bildschirm passend ausgerichtet, um die Trägersubstanz zu dynamisieren.

Das Computerprogrammprodukt kann z.B. in Form einer sogenannten Smartphone-App auf dem Smartphone installiert sein.

Sämtliche Ausführungsformen der vorliegenden Erfindung bieten den Vorteil, dass homöopathische Wirkstoffe in Form analoger homöopathischer Wirkstoffe dezentral, beispielsweise im eigenen Haushalt, von Laien hergestellt werden können. Die Herstellung der analogen homöopathischen Wirkstoffe ist einfach und preiswert, da weder ein Verdünnen und Verschütteln durchgeführt, noch eine Ursprungssubstanz eingesetzt werden muss.

Weiter bieten sämtliche Ausführungsformen der vorliegenden Erfindung den Vorteil, dass beliebige Mischungen klassischer homöopathischer Wirkstoffe als analoge homöopathische Wirkstoffe herstellbar sind.

Details, welche vorstehend in Bezug auf das Herstellungsverfahren beschrieben wurden, gelten auch für die Vorrichtung, den Wirkstoff und das Computerprogrammprodukt. Analoges gilt für Details, welche nur in Bezug auf die Vorrichtung, den Wirkstoff und das Computerprogrammprodukt beschrieben wurden.

Nachstehend wird die Herstellung des analogen homöopathischen Wirkstoffes an einem konkreten Beispiel detailliert beschrieben.

Der Benutzer hat seit einigen Tagen Halsschmerzen sowie eine akute Prellung am Bein. Zur Behandlung beider körperlichen Symptome möchte er nun einen gegen beide Beschwerden gerichteten analogen homöopathischen Wirkstoff herstellen.

Im Vorfeld hat er bereits unbehandelte Globuli, teilweise auch "unarzneiliche Globuli" oder "neutrale Globuli" gekauft, welche beispielsweise aus reiner Saccharose bestehen. Diese Globuli hat er auf einer geeigneten Unterlage, beispielsweise einem Trinkglas-Untersetzer mit Rand oder in einer durchsichtigen Box in einer möglichst dünnen Schicht verteilt.

Der Benutzer hat in der Vergangenheit gerne Belladonna als klassischen homöopathischen Wirkstoff gegen Halsschmerzen eingesetzt, für die Behandlung seiner Prellung wünscht er ebenfalls einen homöopathischen Wirkstoff, hat hier aber keine Präferenz. Die Behandlung soll möglichst einfach sein, d.h. er möchte nicht verschiedene klassische homöopathische Globuli, gegebenenfalls in unterschiedlicher Dosierung und zu unterschiedlichen Zeiten über den Tag verteilt, einnehmen. Daher bedient er sich der vorliegenden Erfindung.

Der Benutzer startet eine auf seinem Smartphone installierte App, also ein Computerprogrammprodukt, welches ihm als Privatperson ohne homöopathische Ausbildung die Herstellung eines analogen homöopathischen Wirkstoffs gemäss einer Ausführungsform der vorliegenden Erfindung in der eigenen Wohnung erlaubt.

Über seinen Smartphone-Bildschirm kann er der App vorgeben, dass der analoge homöopathische Wirkstoff zu Belladonna C30 analog sein soll. Ferner kann er über ein Auswahlmenü umfassend mehrere körperliche Symptome auswählen, dass er eine Prellung am Bein hat, woraufhin die App über eine Internet-Verbindung zu einem Server die Information erhält, dass dieses Symptom mittels des klassischen homöopathischen Wirkstoffs Arnica D12 behandelt werden kann.

Der Benutzer betätigt nach Abschluss beider Eingaben einen Start-Button in Form eines Felds auf seinem Smartphone-Bildschirm, woraufhin ihn das Smartphone mittels Sprachbefehl über den Smartphone-Lautsprecher und/oder mittels Textbefehl, der auf dem Smartphone-Bildschirm angezeigt wird, dazu auffordert, das Handy so auf die vorbereiteten unbehandelten Globuli zu legen, dass der als Strahlungsquelle dienende Smartphone-Bildschirm auf oder über den Globuli liegt. Der Benutzer legt das Smartphone entsprechend dieser Aufforderung auf die ausgebreiteten Globuli oder auf die durchsichtige Box. Beim Starten der Bestrahlung gibt das Smartphone, gesteuert durch die App, ein akustisches Signal aus. Somit weiss der Benutzer, dass das Herstellungsverfahren beginnt. Hat er das Smartphone beim Ertönen des Signals noch nicht korrekt platziert, kann er die App anhalten und von vorne beginnen.

Bei der Programmierung der App wurde unter Mitwirkung eines Homöopathen eine Zuordnungsvorschrift erzeugt und in der App hinterlegt, welche basierend auf den Eingaben des Benutzers eine Strahlungs-Sequenz vorgibt. Im vorliegenden Fall könnte der Smartphone-Bildschirm also über einen gewissen Zeitraum, d.h. über mehrere Sekunden oder Minuten hinweg, in verschiedenen Farben aufleuchten. Die Parameter dieser Sequenz, d.h. die Dauer der aufeinander folgenden Zeitabschnitte sowie die Farbe und die Helligkeit dieser Farbe während der einzelnen Zeitabschnitte, werden durch die Zuordnungsvorschrift eindeutig bestimmt.

Wurde das Smartphone beim Ertönen des oben beschriebenen Signals korrekt platziert, muss der Benutzer lediglich abwarten, bis das Verfahren beendet ist. Dies ist üblicherweise spätestens nach wenigen Minuten der Fall, woraufhin ein weiteres akustisches Signal ertönt, welches dem Benutzer signalisiert, dass das Verfahren abgeschlossen wurde. Er kann sein Smartphone dann wieder aufnehmen, die zuvor unbehandelten Globuli wurden nun durch die Bestrahlung dynamisiert und liegen als analoger homöopathischer Wirkstoff vor.

Soll die Dynamisierung alternativ oder zusätzlich mittels Schallwellen erfolgen, so kann die Ohrmuschel des Smartphones alternativ oder zusätzlich zum Smartphone-Bildschirm zum Einsatz kommen. Wird die Ohrmuschel des Smartphones zur Beaufschlagung der Trägersubstanz mit Schallwellen benutzt, so läuft das Verfahren im Übrigen so ab, wie vorstehend mit Hinblick auf dem Smartphone-Bildschirm beschrieben.

Zusätzlich oder anstelle der Ohrmuschel kann auch ein anderer Lautsprecher des Smartphones, welcher sich beispielsweise seitlich am Smartphone befinden kann, zur Beschallung eingesetzt werden.

Alternativ oder zusätzlich zur Bestrahlung mittels des Smartphone-Bildschirms kann weiterhin ein mit dem Smartphone gekoppelter Lautsprecher (oft als "Bluetooth-Lautsprecher" bezeichnet) zum Einsatz kommen, der die Dynamisierung der unbehandelten Globuli mit Schallwellen bewirken kann. Hierfür kann der Bluetooth-Lautsprecher nahe der unbehandelten Globuli oder auf den Globuli platziert werden. Im Übrigen verläuft die Dynamisierung mittels Schallwellen analog wie vorstehend mit Hinblick auf die Strahlung aus dem Smartphone-Bildschirm beschrieben.

## Patentansprüche

1. Verfahren zur Herstellung eines analogen homöopathischen Wirkstoffes aus einer Trägersubstanz, das Verfahren umfassend folgende Schritte
- Bereitstellen der Trägersubstanz
- Dynamisierung der Trägersubstanz umfassend ein Aufbringen einer elektromagnetischen Strahlung und/oder ein Aufbringen von Schallwellen auf die Trägersubstanz.

2. Verfahren nach Anspruch 1, **dadurch gekennzeichnet, dass** die Dynamisierung der Trägersubstanz aus dem Aufbringen der elektromagnetischen Strahlung und/oder der Schallwellen besteht.

3. Verfahren nach einem der vorhergehenden Ansprüche, **dadurch gekennzeichnet, dass** die elektromagnetische Strahlung geeignet ist, vom Menschen als Farbe wahrgenommen zu werden, und/oder dass die Schallwellen geeignet sind, vom Menschen als Ton wahrgenommen zu werden.

4. Verfahren nach einem der vorhergehenden Ansprüche, **dadurch gekennzeichnet, dass** die elektromagnetische Strahlung und/oder die Schallwellen in einer Sequenz aufgebracht werden, wobei eine solche Sequenz zumindest zwei Zeitabschnitte umfasst, während denen die elektromagnetische Strahlung und/oder die Schallwellen aufgebracht werden, und wobei sich die elektromagnetische Strahlung und/oder die Schallwellen, welchen in diesen Zeitabschnitten aufgebracht werden, im Hinblick auf zumindest einen Parameter unterscheiden.

5. Verfahren nach einem der vorhergehenden Ansprüche, **gekennzeichnet durch** folgende Schritte:
- die elektromagnetische Strahlung und/oder die Schallwellen, welche durch zumindest einen Parameter charakterisiert sind, werden für die Dynamisierung ausgewählt;
- die Dynamisierung der Trägersubstanz erfolgt durch Aufbringen der elektromagnetischen Strahlung und/oder der Schallwellen, welche durch den zumindest einen Parameter charakterisiert sind.

6. Verfahren nach Anspruch 5, **dadurch gekennzeichnet, dass** vor der Auswahl folgende Schritte ausgeführt werden:
- Empfangen einer Eingabe eines Benutzers, die Eingabe umfassend zumindest eine Information,
- die elektromagnetische Strahlung und/oder die Schallwellen, welche durch zumindest einen Parameter charakterisiert sind, werden basierend auf der Eingabe des Benutzers für die Dynamisierung ausgewählt.

7. Verfahren nach Anspruch 6, **dadurch gekennzeichnet, dass** die Eingabe eine Information umfasst, welche zumindest einen klassischen oder analogen homöopathischen Wirkstoff und/oder zumindest eine Verdünnung und/oder zumindest ein körperliches Symptom und/oder zumindest einen Namen einer Zubereitung umfassend einen klassischen homöopathischen Wirkstoff und/oder zumindest einen Namen eines Herstellers einer solchen Zubereitung betrifft.

8. Verfahren nach einem der Ansprüche 1 bis 7, **dadurch gekennzeichnet, dass** die elektromagnetische Strahlung und/oder die Schallwellen von einem mit Computer-Funktionalitäten ausgestatteten Gerät, beispielsweise von einem Smartphone, oder einem System umfassend ein solches Gerät auf die Trägersubstanz aufgebracht werden.

9. Vorrichtung, welche eingerichtet ist, ein Verfahren gemäss zumindest einem der Ansprüche 1 bis 8 durchzuführen.

10. Vorrichtung gemäss Anspruch 9, **dadurch gekennzeichnet, dass** die Vorrichtung ein mit Computer-Funktionalitäten ausgestattetes Gerät, insbesondere ein Smartphone ist.

11. System umfassend eine Vorrichtung gemäss Anspruch 10.

12. System umfassend eine tragbare Vorrichtung nach Anspruch 11 sowie eine Halterung, welche eingerichtet ist, die tragbare Vorrichtung in einem vorbestimmten Abstand zu einer in ihrer Nähe platzierten Trägersubstanz zu fixieren.

13. Computerprogrammprodukt umfassend Instruktionen, die, wenn sie von einem mit Computer-Funktionalitäten ausgestatteten System oder Gerät ausgeführt werden, bewirken, dass dieses die Schritte gemäss zumindest einem der Ansprüche 1 bis 8 ausführt.

14. Analoger homöopathischer Wirkstoff, hergestellt gemäss einem der Ansprüche 1 bis 8.
